# EUROPEAN PATENT SPECIFICATION

(11) **EP 3 028 025 B1**
(45) Date of publication and mention of the grant of the patent: **17.04.2019**
(21) Application number: 14744114.1
(22) Date of filing: 28.07.2014
(51) Int. Cl.: G01M 3/16

(54) **LEAK DETECTION SENSOR FOR A STOMA POUCH AND A PROCESS FOR MAKING SAME**
SENSOR ZUM ERKENNEN VON UNDICHTIGKEITEN UND HERSTELLUNGSVERFAHREN
CAPTEUR DE DÉTECTION DE FUITE POUR POCHE DE STOMIE ET PROCÉDÉ DE FABRICATION

(30) Priority: 31.07.2013 GB 201313721
(43) Date of publication of application: 08.06.2016
(73) Proprietor: University Of Ulster, County Londonderry BT52 1SA (GB)
(72) Inventor: DAVIS, James, Newtownabbey County Antrim BT37 0QB (GB); PHAIR, Jolene, Newtownabbey County Antrim BT37 0QB (GB); MCDONALD, Damian, Newtownabbey County Antrim BT37 0QB (GB)
(74) Representative: FRKelly
(86) International application number: PCT/EP2014/066140
(87) International publication number: WO 2015/014774

(56) References cited:
- US-A- 3 270 105

## Description

### FIELD OF THE INVENTION

This invention relates to a leak detection sensor for a stoma pouch and a process for making same.

### BACKGROUND OF THE INVENTION

In the treatment of certain medical conditions, such as cancer of the colon, it is known to establish an opening in the body for the discharge of body wastes. Such opening is typically known as an ostomy. There are three types, namely urostomy, ileostomy and colostomy, depending upon where such opening is located. A stoma is an outlet from the intestine through the abdominal wall. Such openings are typically connected to an exchangeable bag for receiving the body wastes, such bag typically being known as a stoma pouch. The stoma pouch is typically connected to the ostomy via a flange attachable to the skin .around the stoma by means of an adhesive layer. The pouch may be detachable from the flange to permit replacement of the pouch.

The adhesive layer of the flange gradually loses its effectiveness over time, leading to leakages having unpleasant consequences. Therefore it is desirable to have a reliable and cost effective system capable of detecting the leakage of fluid beneath the adhesive layer of the flange of a stoma pouch to warn the patient that the pouch requires replacement.

There are approximately 120,000 people in the UK with some form of "ostomy" and it is estimated that 21,000 operations to create them are performed each year. The US has, as expected, a far higher number with more than 650,000 ostomates. The cost to the NHS alone for the cost of accessories (pouches, flanges etc.) for the effective management ranges from £780 to £2300 per person per year with the total annual spend in the region of £200m. The most pressing complaint raised by patients is not the inconvenience of the pouch system but rather the consequences of its failure, especially in social settings. There is a tremendous psychological burden on the patient when trying to lead a normal, proactive life where there is a constant fear of the pouch leaking and the associated embarrassment. An object of the present invention is to provide an early warning system which will alert the patient to the impending leak and give them sufficient time to take preventative action and thus avoid the debilitating scenario of having to deal with a pouch failure in public. US 3 270 105 discloses a known leak detection sensor.

### SUMMARY OF THE INVENTION

According to the present invention there is provided a method of making a leak detection sensor comprising providing a pair of electrodes each having an electrically insulating coating thereon formed from a thermopolymer, and creating fractures in the coating of the electrodes having a breadth of less than 500nm, more preferably 100nm or less, through which fluid may permeate to enable current to flow between the electrodes when exposed to an electrolyte wherein said step of creating fractures in the coating of the electrodes may comprise creating successive troughs at spaced intervals in the coating of the electrodes by laser ablation of the coating, exposing the surface of the respective electrode in said troughs, whereby the transmission of heat along the length of the respective electrode during the creation of a respective trough results in the partial melting of the coating surrounding a previously ablated adjacent trough which recovers the previously exposed surface of the electrode, fractures arising in the remelted coating as it solidifies as a consequence of the previous thermal degradation of the coating.

The electrodes may comprise a pair of wires, said method preferably comprising the step of intertwining said wires following creation of said fractures the respective coating thereof.

The method may comprise the further step of intertwining an absorbent wick with said wires.

The laser ablated troughs may have a length of approximately 25 microns and may be created at a spacing of approximately 2mm.

### BRIEF DESCRIPTION OF THE DRAWINGS

An embodiment of the present invention will now be described, by way of example only, with reference to the accompanying drawings, in which:-
Figure 1 shows a stoma pouch leak detection assembly made in accordance with an embodiment of the present invention;
Figure 2 is an electron microscope image of a portion of the leak detection sensor of the assembly of Figure 1;
Figure 3 is a higher magnification image of the surface of the sensor showing the nanoscale fractures formed in the surface of the insulating coating of the wires thereof.

### DETAILED DESCRIPTION OF THE DRAWINGS

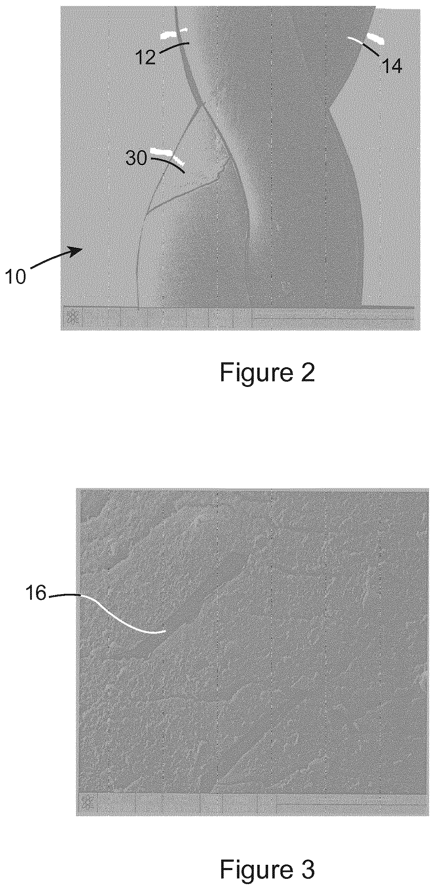

Made in accordance with an embodiment of the present invention, a stoma pouch leak detection assembly comprises a leak detection sensor 10 comprising two intertwined copper wires 12,14, each wire being coated with a thermopolymer, such as polyvinylacetate or polyester, to define an electrically insulating coating, the coating of the wires being treated by laser ablation to create nanoscale fractures 16 (see Figure 3) in the insulating coating of the wires 12,14 through which fluid may permeate, allowing conduction between the wires and thus detection of leaks. The term "nanoscale fractures" used herein is hereby defined as fractures having a breadth of less than 500nm, more 100nm or less. The fractures may have a length of between 500nm and 5 microns.

The sensor 10 may also include a porous wick (not shown) intertwined with the wires 12,14. This may increase the sensitivity of the device such that, upon a leak emerging at a discrete point, the fluid is transported along the wire network by capillary action and thus contacts a greater proportion of nanoscale fractures 16, increasing the sensitivity of the sensor 10.

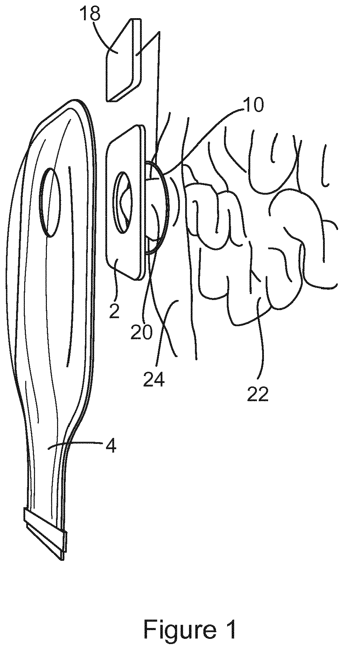

As shown in Figure 1, the sensor 10 is shaped and dimensioned to encircle an opening in a flange 2 of a stoma pouch 4 to sit between the skin 24 and an adhesive layer of the flange 2 when the flange 2 is located over a stoma 20 protruding through the skin 24 from the small intestine 22, such that fluid leaking behind the adhesive layer may be detected by the sensor 10, triggering a discrete alarm device 18.

A process of laser ablation of the insulating coating covering the intertwined copper wires 12,14 of the sensor 10 creates nanoscale fractures in the coating through which fluid may permeate to enable the detection of leaks between the adhesive layer and the skin of the patient without the risk of short circuits between the wires 12,14.

The leak detection sensor 10 made in accordance with the present invention can be applied to conventional stoma pouch and thereby alert the wearer to the early onset of a potential leak. The leak detection sensor 10 may be connected to an alarm device or detector 18, which may comprise a conductivity circuit connected to a vibration alarm or other user perceptible alert, such as an audible and/or visually perceptible alarm. The leak detection sensor 10 defines a loop encircling the opening in the flange 2 of the stoma pouch 4 to be located between the skin of the patient and the adhesive layer of the flange 2.

Fluid draining into the pouch has a tendency to undermine the adhesive layer of the flange 2 and, if left untreated, can lead to the failure of the flange 2 and the release of bowel or bladder contents. The leak detection sensor 10 may be located on or adjacent the adhesive layer, near the periphery of the flange opening such that, upon contact with fluid leaking past the adhesive layer and coming into contact with the sensor, the fluid, defining an electrolyte, completes the detection circuit and activates the vibration alarm, which may provide a discrete alarm signal to inform the patient that the pouch needs to be changed.

The sensor wires 12,14 define two electrodes, preferably in the form of intertwined polyvinylacetate copper wires coated with an electrically insulating polymer coating, wherein the coating has been laser treated to create nanoscale factures 16 in the insulating coating, through which the stoma fluid can permeate and hence enable charge transfer between the two wires and thereby raise the alarm. The bulk of the coating however is complete, preventing circuit shorting and avoiding direct contact of the copper wire with the skin and overcomes issues with biocompatibility and peristomal irritation. Acid from the stoma fluid can corrode uncoated copper wires if placed directly in contact with the skin and thus lead to copper ions being mobilised. The copper ions could lead to skin complications and potential copper poisoning. The provision of the coating on the wires 12,14 prevents this.

The insulating coating may comprise polyvinylacetate or any other suitable thermopolymer, such as polyimide, polyurethane or polyester.

By using laser ablation to create nanoscale fractures 16 in the coating, such cracks allow the permeation of the stoma fluid through to the underlying copper, hence enabling the charge to transfer between the two electrodes. However, the coating remains intact and thus prevents the copper from coming into direct contact with one another and with the patient's skin, preventing short circuits. The sensor itself must lie between the skin and the flange and thus the provision of an insulating coating eases issues of biocompatibility. While the stoma fluid can still corrode the copper, the surface area of copper exposed via the nanoscale fractures is minimal and transport through the factures of the mobilised copper ion would be significantly restricted (a diffusion based transport process) and thus would be unlikely to cause any irritation issues.

In a preferred embodiment the nanoscale fractures 16 in the insulating coating may be created by rastering across the polymer coated copper wire using a 25W carbon dioxide laser to create 25 micron troughs at intervals of 2mm along the length of the sensor and which partially exposes the underlying metal. The polyvinylacetate (PVAc) coating is a thermopolymer and the laser processing results in the partial ablation of the polymer. A critical issue however is the thermal conductivity of the underlying copper. After removing one segment the laser may be repositioned to remove another. The transmission of heat along the length of the wire however results in the partial melting of the polymer surrounding the previously ablated segment which recovers the metal with molten polymer. When cool, nanoscale fractures 16 arise as a consequence of the previous thermal degradation.

The copper wires 12,14 may then be intertwined with a polyester thread (to create a wick) and the final assembly may serve as a two electrode sensor 10 which can be placed onto the pouch flange around the periphery of the flange opening. The adhesive coating of the pouch flange enables easy positioning of the sensor 10. The flange plus sensor can then be placed directly over the stoma onto the skin and the sensor connections attached to the detector 18. It is envisaged that the wire 12,14 may be intertwined prior to the process of laser ablation.

As fluid is released form the stoma, some will begin to seep under the flange and will gradually undermine the adhesive seal. The detector 18 is activated when the fluid reaches the sensor 10 and effectively completes the circuit. The degree of warning can be controlled by the user through the placement of the sensor 10 with respect to the adhesive layer around the flange opening. The closer to the flange periphery, the shorter the period before warning and flange failure.

Figure 2 comprises a scanning electron micrograph highlighting the morphology of the sensor structure. The troughs 30 created by the laser are clearly visible in Figure 2 but it is important to note that the ablated portion does not reveal the underlying copper. The transmission of heat along the length of the wire however results in the partial melting of the polymer surrounding the previously ablated segment which recovers the metal with molten polymer. When cool, the nanoscale fractures 16 are created in the coating as a consequence of the previous thermal degradation of the coating. The nanoscale fractures 16 can be seen in greater detail in Figure 3.

The operation of the sensor 10 made in accordance with the present invention has been tested ex vivo with stoma fluid. There is a high electrolyte concentration in the stoma output which yields a positive response at the detector.

The crux of the issue relates to the design of a conductivity sensor that can be mounted onto a biomedical flange which can provide 360° detection of impending leaks. The electrodes of the sensor can be intertwined into a single loop using low cost material and which enables continuous monitoring without inducing skin irritation.

The detector assembly itself can be re-usable, with the sensor 10 comprising a low cost disposable component. Crucially, the sensor can integrated within existing pouch systems without alteration. The flexible nature of the sensor 10 allows it to be tailored to a wide range of stoma pouches (independent of shape and size) by the patient themselves.

## Claims

1. A method of making a leak detection sensor (10) comprising providing a pair of electrodes (12,14) each having an electrically insulating coating thereon formed from a thermopolymer, and creating fractures (16) in the coating of the electrodes having a breadth of less than 500nm through which fluid may permeate to enable current to flow between the electrodes when exposed to an electrolyte **characterised in that** said step of creating fractures (16) in the coating of the electrodes (12,14) comprises creating successive troughs at spaced intervals in the coating of the electrodes (12,14) by laser ablation of the coating, exposing the surface of the respective electrode (12,14) in said troughs, whereby the transmission of heat along the length of the respective electrode (12,14) during the creation of a respective trough results in the partial melting of the coating surrounding a previously ablated adjacent trough which recovers the previously exposed surface of the electrode (12,14), said fractures (16) arising in the remelted coating as it solidifies as a consequence of the previous thermal degradation of the coating.

2. A method as claimed in claim 1, wherein said fractures (16) have a breadth of 100nm or less.

3. A method as claimed in claim 1 or claim 2, wherein said electrodes comprise a pair of wires (12,14), said method comprising the step of intertwining said wires (12,14) following creation of said fractures (16) the respective coating thereof.

4. A method as claimed in claim 3, comprising intertwining an absorbent wick with said wires (12,14).

5. A method as claimed in any preceding claim, wherein said troughs have a length of approximately 25 microns and are created at a spacing of approximately 2mm.

## Patentansprüche

1. Verfahren zum Herstellen eines Leckerkennungssensors (10), umfassend das Bereitstellen eines Paars Elektroden (12, 14), die jeweils eine aus einem Thermopolymer gebildete elektrisch isolierende Beschichtung darauf aufweisen, und das Erzeugen von Rissen (16) in der Beschichtung der Elektroden, die eine Breite von weniger als 500 nm aufweisen, durch die Fluid dringen kann, um zu ermöglichen, dass Strom zwischen den Elektroden fließt, wenn sie einem Elektrolyten ausgesetzt sind, **dadurch gekennzeichnet, dass** der Schritt des Erzeugens von Rissen (16) in der Beschichtung der Elektroden (12, 14) Folgendes umfasst: Erzeugen aufeinanderfolgender voneinander beabstandeter Rillen in der Beschichtung der Elektroden (12, 14) durch Laserablation der Beschichtung, Freilegen der Oberfläche der jeweiligen Elektrode (12, 14) in den Rillen, wodurch die Übertragung von Wärme entlang der Länge der jeweiligen Elektrode (12, 14) während der Erzeugung einer jeweiligen Rille zum partiellen Schmelzen der eine zuvor abladierte benachbarte Rille umgebenden Beschichtung führt, was die zuvor freigelegte Oberfläche der Elektrode (12, 14) wieder verdeckt, wobei die Risse (16) als Folge des vorherigen thermischen Abbaus der Beschichtung in der erneut geschmolzenen Beschichtung auftreten, während sie erstarrt.

2. Verfahren nach Anspruch 1, wobei die Risse (16) eine Breite von 100 nm oder weniger aufweisen.

3. Verfahren nach Anspruch 1 oder Anspruch 2, wobei die Elektroden ein Paar Drähte (12, 14) umfassen, wobei das Verfahren den Schritt des Verflechtens der Drähte (12, 14) nach dem Erzeugen der Risse (16) in der jeweiligen Beschichtung derselben umfasst.

4. Verfahren nach Anspruch 3, umfassend das Verflechten eines saugfähigen Dochts mit den Drähten (12, 14).

5. Verfahren nach einem der vorangehenden Ansprüche, wobei die Rillen eine Länge von ungefähr 25 Mikrometern aufweisen und in einem Abstand von ungefähr 2 mm erzeugt werden.

## Revendications

1. Procédé de fabrication d'un capteur de détection de fuite (10) comprenant la fourniture d'une paire d'électrodes (12, 14) ayant chacune un revêtement électriquement isolant sur celles-ci formé à partir d'un thermopolymère, et la création de fractures (16) dans le revêtement des électrodes ayant une largeur inférieure à 500 nm par lesquelles du fluide peut passer pour permettre à un courant de circuler entre les électrodes lorsqu'elles sont exposées à un électrolyte **caractérisé en ce que** ladite étape de création de fractures (16) dans le revêtement des électrodes (12, 14) comprend la création de dépressions successives à des intervalles espacés dans le revêtement des électrodes (12, 14) par ablation laser du revêtement, exposant la surface de l'électrode respective (12, 14) dans lesdites dépressions, ce par quoi la transmission de chaleur le long de la longueur de l'électrode respective (12, 14) lors de la création d'une dépression respective conduit à la fusion partielle du revêtement entourant une dépression adjacente ayant précédemment subi une ablation qui recouvre la surface précédemment exposée de l'électrode (12, 14), lesdites fractures (16) survenant dans le revêtement refondu à mesure qu'il se solidifie en conséquence de la dégradation thermique précédente du revêtement.

2. Procédé tel que revendiqué dans la revendication 1, dans lequel lesdites fractures (16) ont une largeur de 100 nm ou moins.

3. Procédé tel que revendiqué dans la revendication 1 ou la revendication 2, dans lequel lesdites électrodes comprennent une paire de fils (12, 14), ledit procédé comprenant l'étape consistant à entremêler lesdits fils (12, 14) comme suite à la création desdites fractures (16) dans le revêtement respectif de celles-ci.

4. Procédé tel que revendiqué dans la revendication 3, comprenant le fait d'entremêler une mèche absorbante avec lesdits fils (12, 14).

5. Procédé tel que revendiqué dans l'une quelconque des revendications précédentes, dans lequel lesdites dépressions ont une longueur d'approximativement 25 microns et sont créées à un espacement d'approximativement 2 mm.
